# EUROPEAN PATENT SPECIFICATION

(11) **EP 2 353 632 B1**
(45) Date of publication and mention of the grant of the patent: **19.12.2012**
(21) Application number: 10153046.7
(22) Date of filing: 09.02.2010
(51) Int. Cl.: A61M 25/10

(54) **Medical device for local drug delivery**
Medizinisches Gerät zur lokalen Arzneimittelverabreichung
Dispositif médical pour l'administration locale de médicament

(43) Date of publication of application: 10.08.2011
(73) Proprietor: Sanofi-Aventis Deutschland GmbH, 65929 Frankfurt am Main (DE)
(72) Inventor: Gille, Andreas, 3031 Ascot Vale, Victoria (AU); Schäfer, Matthias, 65926 Frankfurt am Main (DE)

(56) References cited:
- EP-A2- 0 581 708
- US-A- 5 599 301
- US-A1- 2003 101 800

## Description

The present invention relates to a medical device for the local delivery of a drug.

In particular, the present invention relates to a balloon catheter adapted for the local delivery of a drug, especially for the use in the treatment of myocardial infarct or other occlusions of body vessels or in the perfusion of organs or tissues to be explanted.

Such medical devices for the local and targeted delivery of a drug are especially useful in the treatment of numerous diseases which do not affect the entire organism at the same time but are restricted to specific tissues, often even limited to individual tissue areas or organ paris. Pharmacotherapy of such diseases like tumor, joint and vascular diseases, generally is affected by oral or intravenous administration of drugs that can cause undesired side effects by this systemic uptake in healthy tissues and organs. Selective administration by direct injection into the desired tissue or supply via a catheter to the blood vessels that feed the diseased tissue is an option. However, in these methods of local application the problem of a too short time period where the drugs an efficacious in the targeted area before being washed out arises. Thus, the drugs are insufficiently extracted in the targeted area.

In more recent times, it is known to deliver drugs to a local area of a body vessel or an organ part by catheters which can be coated with the drug or which are equipped with special devices for injecting drugs into the vessel wall like needles or a perforation of the catheter wall. However, the known catheters equipped for a local drug delivery often lack the possibility to control the release and the dosage of the drug especially in treatments that comprise a repeated or sequential treatment of a targeted area.

US 5 599 301 A discloses a motor control system for an automatic catheter inflation system. The system comprises a balloon catheter which is automatically inflated and deflated according to a preselected program of a controller. The controller can adjust the inflation and/or deflation of the balloon in dependence of a sensor signal. The sensor signal described represents the fluid pressure of the fluid pumped into the catheter balloon which is also an indicator for the balloon pressure itself. Moreover, catheters having a perforated or other specialized type of balloon for delivery of drugs to the site of the stenosis can be comprised.

It is an objective of the present invention to provide an improved medical device adapted for the local delivery of a drug, such as pharmaceutically and/or therapeutically active agents. Particularly, it is an objective to provide a possibility of a better control of the release and dosage of the drug even in a repeated or sequential treatment.

In order to achieve this objective, the present invention provides a medical device adapted for local drug delivery comprising:
a balloon catheter comprising means for a local delivery of a drug, functionally connected to
at least one controllable automatically driven pump,
at least one pressure sensor and
a control unit for controlling the pump depending on a pressure signal of the pressure sensor wherein
at least one pressure sensor is placed on the surface of the balloon.

The balloon catheter used in the invention can be for example an infusion catheter, and especially an angioplasty balloon catheter. The balloon catheter can comprise at least one dilatation balloon disposed on a flexible tubular catheter body. The dilatation balloon is preferably made from a non-elastomeric thin walled polymer material such as polyvinylchloride, polyethylene, various derivatives thereof or other suitable film-forming material, capable of withstanding pressures of ≥ 100 psi, or ≥ 150 psi, without being damaged and without significant or undue stretch or deformation when inflated into the thin-walled balloon. Suitable materials are generally known to those skilled in the art of angioplasty. The material of the dilatation balloon provides for a stable balloon expansion even under high inflation pressure.

There is at least one pressure sensor functionally connected to the balloon catheter according to the present invention. The pressure sensor can be placed on the catheter itself. According to the invention at least one pressure sensor is placed on the surface of the balloon.

The controllable automatically driven pump in the present invention is functionally connected to the balloon catheter; thereby for instance, providing for an accurate controlled inflation and deflation of the balloon. Therefore the pump can be used to start, to stop and/or to reduce the blood flow. In another aspect, the controlled automatically driven pump functionally connected to the balloon catheter is a fluid pump furnishing a drug comprising fluid to the local area to be treated which is separate and independent from the inflation pump of the balloon. However, the control of this pump can still be provided in dependence to the inflation pressure of the balloon.

This allows for an even more exact regulation of a fluid flow dependent on the inflation pressure applied to the balloon and the resulting expansion thereof. Thus, the factors of human inconsistency and error in both the dilatation phase and post conditioning treatment can be reduced or avoided. In particular, the fluid flow is the flow of an inflation fluid which can comprise a drug in case the balloon is a porous or microporous balloon.

With the control unit of the invention the pump can be controlled depending on a pressure signal of the pressure sensor. For example, different pre-programmed series of sequential inflation and deflation of the balloon in a targeted extent resulting in full sealing or reduced low pressure blood flow at a time can be stored in the control unit and can thus be chosen by the medical person to fit to the needs of the patient. Like that, a high standard quality of the treatment can be achieved. Thus, the factors of human inconsistency and error in the treatment can be reduced or avoided. Thereby it is possible to provide an improved automated and computer controlled treatment.

The control unit of the invention may comprise a micro processor. The control unit may advantageously be capable of storing data, for example relevant parameters of the performed procedure. Therefore, this embodiment of the present invention can give additional information about the quality of the treatment to the medical practitioner and helps to further improve the local delivery of a drug in a bodily vessel or in a section of an organ, for instance in post conditioning treatment, as medical method. Standardization and reproduction of optimized performance with the obtained reliable data of the medical treatment are made possible. Even the performance of different medical practitioners can be compared and optimized clinical studies are conceivable.

In one aspect of the invention the pump is adapted for the pumping and/or suction of a fluid. The fluid can in one aspect be any fluid used for inflating the balloon like a suitable aqueous solution, for instance saline, sterile water, a perfusate like blood, Ringer's solution, a buffer solution, or a suitable gas like helium or carbon dioxide. In another aspect the fluid can be any perfusate used for perfusion of the targeted local area which preferably comprises a suitable drug.

The term "drug" can comprise any suitable active agent used for a medical treatment of a bodily vessel or part of it. Examples of suitable drugs include tissue protective agents like steroids to suppress localized edema and inflammation, adenosine, anti-oxidants, saline to replace lost intravascular fluid, antagonists of platelet aggregation, thrombolytic agents and anticoagulants. Preferably, the drug is comprised in a pharmaceutically acceptable aqueous carrier.

In another aspect of the invention the pump is drivable at an adjustable pumping rate and/or an adjustable pumping pressure. Like that, the dosage of the locally delivered drug can be very accurately controlled and adapted to the actual needs of the patient even during the application. Additionally, the risk of a so called "jet injury" of the vessel caused by too much pressure by the perfusate introduced in the targeted area of the vessel lumen or by a too rapid introduction of the perfusate can be reduced or even avoided.

According to the invention the pump can be a syringe pump, preferably a programmable syringe pump.

In one aspect of the medical device according to the invention the pressure signal is the inflation pressure of the balloon and/or the perfusion pressure of a pumped fluid.

In one embodiment the medical device according the invention comprises at least one pressure transducer for measuring a physiological parameter and/or pressure signal and for submitting a corresponding signal to the control unit. The signal submission can be achieved by thin electric or optical wires or the signal can be transmitted by wireless communication of the two components.

The physiological parameter can be selected from the group consisting of systemic blood pressure, blood pressure before the balloon catheter, blood pressure behind the balloon catheter, blood flow before the balloon catheter, blood flow behind the balloon catheter and electrocardiogram.

In another aspect of the invention the control unit of the medical device controls the pumping time and/or the pumping volume. Again, a very accurate dosage of the drug at the targeted area of the vessel can be achieved by controlling the additional parameters of pumping time and pumping volume. In particular, the pumping time and pumping volume can be varied over the duration of the whole treatment and a computer programmed series of different times and volumes can be applied, preferably in combination and in dependency to the inflation time and deflation time of the balloon of the catheter.

According to another aspect of the present invention the means for the local delivery of a drug comprise at least one porous or microporous balloon.

In one preferred embodiment of the invention the means for the local delivery of a drug comprise a coating on the balloon. Coating balloons on catheters with drugs according to this invention is particularly useful because there is a frequent need for treatment after blood vessels or other passages in the body were dilated with balloons to prevent stenosis or an occlusion of the lumen created by the pressure of the balloon, to limit tumor growth or to enhance healing processes including the formation of collateral circulation. This can be effectively achieved by drugs that become effective in the immediate vicinity of the balloon surface. The drugs firmly adhere to the balloon while passing through the body vessels on their way to the targeted area until the balloon is inflated and only the contact to the vessel tissue activates the drug release. Beneficially, the drug is absorbed by the contacted tissue and subsequent reperfusion of the area does not rinse off the drug from its target area. According to the present invention the contact time and contact pressure of the balloon to the target tissue can be controlled leading to an exact dosage of the drug without risking either a jet injury of the vessel or an over-extension of the vessel by the application of too much pressure by the balloon.

In one aspect according to the invention the balloon catheter comprises at least two balloons. Like that, by inflation of the two balloons spaced apart a well defined area of the vessel can be determined for the local delivery of a drug with a reduced or even without immediate blood flow. The means for delivery of a drug in this embodiment can comprise an outlet of the tubular body of the catheter placed between the two balloons in fluid connection to a perfusate pump. Like that, the drug can be applied to the sealed off targeted area for a predetermined duration and even drugs that shall not enter the blood cycle can be applied and withdrawn again before the balloons are deflated. This is especially advantageous in the treatment of tumors with cytostatic drugs.

According to this aspect of the invention the means for the local delivery of a drug preferably comprise a separate lumen in the catheter. In the separate lumen a sensor can be placed for controlling the pressure or the flow of the fluid carrying the drug. Thus, an improved control of the dosage is possible. Additionally, the flow of the fluid carrying the drug can be precisely adjusted to the inflation and deflation of the balloons.

In a preferred embodiment of the present invention the medical device can be advantageously used in the treatment of myocardial infarct or other occlusions of body vessels.

Alternatively, the medical device according to the present invention can also advantageously be used in the perfusion of organs or tissues to be explanted.

The invention will now be described, by way of example only, with reference to the accompanying drawing, in which:

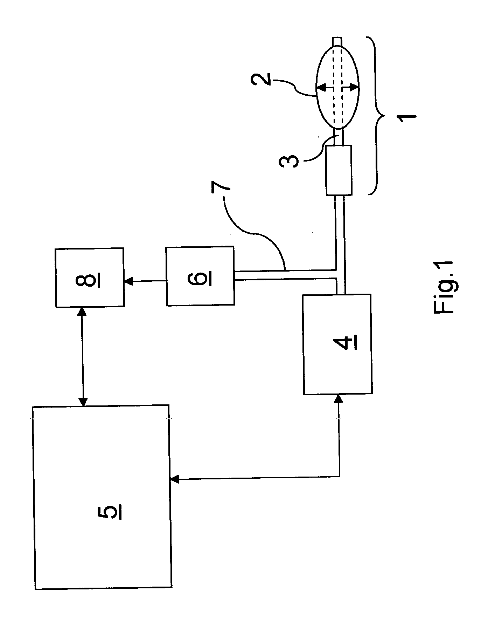

Figure 1 illustrates schematically the components of one embodiment of the medical device according to the invention.

All components and interconnecting elements between the components are simplified in this illustration. According to the invention a balloon catheter 1 is equipped with at least one inflatable and deflatable balloon 2. A first lumen 3 is fluidly connected with the balloon 1 and a fluid or gas is passed through said first lumen 3 to inflate as well as deflate the first balloon. In the presented embodiment the balloon is a porous or microporous balloon which together with the first lumen builds the means for delivery of a drug. The drug is comprised in the fluid which is pumped by the pump for inflation of the balloon and at the same time for the local delivery of the drug by passing through the pores of the balloon. The catheter comprises a proximal and a distal end and the proximal end of the catheter 1 is fluidly connected to a pump 4. The pump 4 is preferably a programmable syringe pump, which can communicate with and can be controlled by a control unit 5. The control unit 5 may regulate and control the pumping rate, pumping volume and/or the pumping time of the pump 4. A pressure transducer 6 according to the shown embodiment is connected in a sealed fluid connection 7 to the pump 4. The pressure transducer 6 may be connected to an AD converter 8 for data translation and for submitting a signal to the control unit 5. According to the submitted signals the inflation of the balloon 1 may be controlled. The pressure measurements can help to determine the exact pressure of the balloon 1, for example to just seal a body vessel from the blood flow without risking injury of the vessel by over-expansion or by applying too much pressure to the vessel. With the control unit 5 and the programmable syringe pump 4 different pre-programmed series of sequential inflation and deflation of the balloon 2 in a targeted extent resulting in full sealing or reduced low pressure blood flow at a time can be performed and can be chosen by the medical practitioner to fit to the needs of the patient. The balloon inflation and therefore also the degree of obstruction of the blood flow can be controlled periodically or continuously based on the pressure signal. Like that, a high standard quality of the treatment can be achieved. Thus, the factors of human inconsistency and error in the treatment can be reduced or avoided. Thereby it is possible to provide an improved automated and computer controlled treatment.

## Claims

1. A medical device adapted for local drug delivery comprising:
a balloon catheter (1) comprising means for a local delivery of a drug functionally connected to
at least one controllable automatically driven pump (4),
at least one pressure sensor (6) and
a control unit (5) for controlling the pump depending on a pressure signal of the pressure sensor (6),
**characterized in that**
at least one pressure sensor (6) is placed on the surface of the balloon (2).

2. The medical device according to claim 1 **characterized in that** the pump (4) is adapted for the pumping and/or suction of a fluid.

3. The medical device according to claim 1 or 2 **characterized in that** the pump (4) is drivable at an adjustable pumping rate and/or an adjustable pumping pressure.

4. The medical device according to one of the claims 1 to 3 **characterized in that** the pump is a syringe pump (4).

5. The medical device according to claim 4 **characterized in that** the syringe pump (4) is a programmable syringe pump.

6. The medical device according to one of the preceding claims **characterized in that** the automatically driven pump (4) is a fluid pump for pumping a drug comprising fluid and the medical device further comprises an inflation pump which is separate and independent from the fluid pump (4).

7. The medical device according to one of the preceding claims **characterized in that** the pressure signal is the inflation pressure of the balloon (2) and/or the perfusion pressure of a pumped fluid.

8. The medical device according to one of the preceding claims **characterized in that** the medical device comprises at least one pressure transducer for measuring a physiological parameter and/or pressure signal and for submitting a corresponding signal to the control unit (5).

9. The medical device according to claim 8 **characterized in that** the physiological parameter is selected from the group consisting of systemic blood pressure, blood pressure before the balloon catheter (1), blood pressure behind the balloon catheter (1), blood flow before the balloon catheter (1), blood flow behind the balloon catheter (1) and electrocardiogram.

10. The medical device according to one of the preceding claims **characterized in that** the control unit (5) controls the pumping time and/or the pumping volume.

11. The medical device according to one of the preceding claims **characterized in that** the means for local delivery of a drug comprise at least one porous or microporous balloon (2).

12. The medical device according to one of the preceding claims **characterized in that** the balloon catheter including means for local delivery of a drug comprises a coating on the balloon (2).

13. The medical device according to one of the preceding claims **characterized in that** the balloon catheter (1) comprises at least two balloons.

14. The medical device according to claim 12 **characterized in that** the means for local delivery of a drug comprise a separate lumen in the catheter.

15. The medical device according to one of the preceding claims for use in the treatment of myocardial infarct or other occlusions of body vessels.

16. The medical device according to one of the preceding claims 1 to 14 for use in the perfusion of organs or tissues to be explanted.

## Patentansprüche

1. Medizinische Vorrichtung, die für eine lokale Arzneimittelverabreichung ausgelegt ist und Folgendes umfasst:
einen Ballonkatheter (1), der Mittel für eine lokale Verabreichung eines Arzneimittels enthält, die funktionstechnisch verbunden sind mit
wenigstens einer steuerbaren, automatisch angetriebenen Pumpe (4),
wenigstens einem Drucksensor (6) und
einer Steuereinheit (5), um die Pumpe in Abhängigkeit von einem Drucksignal des Drucksensors (6) zu steuern,
**dadurch gekennzeichnet, dass**
wenigstens ein Drucksensor (6) auf der Oberfläche des Ballons (2) angeordnet ist.

2. Medizinische Vorrichtung nach Anspruch 1, **dadurch gekennzeichnet, dass** die Pumpe (4) dafür ausgelegt ist, ein Fluid zu fördern und/oder anzusaugen.

3. Medizinische Vorrichtung nach Anspruch 1 oder 2, **dadurch gekennzeichnet, dass** die Pumpe (4) mit einer einstellbaren Pumprate und/oder einem einstellbaren Pumpdruck angetrieben werden kann.

4. Medizinische Vorrichtung nach einem der Ansprüche 1 bis 3, **dadurch gekennzeichnet, dass** die Pumpe eine Spritzenpumpe (4) ist.

5. Medizinische Vorrichtung nach Anspruch 4, **dadurch gekennzeichnet, dass** die Spritzenpumpe (4) eine programmierbare Spritzenpumpe ist.

6. Medizinische Vorrichtung nach einem der vorhergehenden Ansprüche, **dadurch gekennzeichnet, dass** die automatisch angetriebene Pumpe (4) eine Fluidpumpe ist, um ein Arzneimittel enthaltendes Fluid zu fördern, und die medizinische Vorrichtung ferner eine Aufblaspumpe enthält, die von der Fluidpumpe (4) getrennt und unabhängig ist.

7. Medizinische Vorrichtung nach einem der vorhergehenden Ansprüche, **dadurch gekennzeichnet, dass** das Drucksignal der Aufblasdruck des Ballons (2) und/oder der Perfusionsdruck eines geförderten Fluids ist.

8. Medizinische Vorrichtung nach einem der vorhergehenden Ansprüche, **dadurch gekennzeichnet, dass** die medizinische Vorrichtung wenigstens einen Druckwandler enthält, um einen physiologischen Parameter und/oder ein Drucksignal zu messen und um ein entsprechendes Signal an die Steuereinheit (5) auszugeben.

9. Medizinische Vorrichtung nach Anspruch 8, **dadurch gekennzeichnet, dass** der physiologische Parameter aus der Gruppe gewählt ist, die besteht aus systemischem Blutdruck, Blutdruck vor dem Ballonkatheter (1), Blutdruck hinter dem Ballonkatheter (1), Blutfluss vor dem Ballonkatheter (1), Blutfluss hinter dem Ballonkatheter (1) und Elektrokardiogramm.

10. Medizinische Vorrichtung nach einem der vorhergehenden Ansprüche, **dadurch gekennzeichnet, dass** die Steuereinheit (5) die Pumpzeit und/oder das Pumpvolumen steuert.

11. Medizinische Vorrichtung nach einem der vorhergehenden Ansprüche, **dadurch gekennzeichnet, dass** die Mittel zum lokalen Verabreichen eines Arzneimittels wenigstens einen porösen oder mikroporösen Ballon (2) enthalten.

12. Medizinische Vorrichtung nach einem der vorhergehenden Ansprüche, **dadurch gekennzeichnet, dass** der Ballonkatheter, der Mittel für die lokale Verabreichung eines Arzneimittels enthält, eine Beschichtung auf dem Ballon (2) aufweist.

13. Medizinische Vorrichtung nach einem der vorhergehenden Ansprüche, **dadurch gekennzeichnet, dass** der Ballonkatheter (1) wenigstens zwei Ballone umfasst.

14. Medizinische Vorrichtung nach Anspruch 12, **dadurch gekennzeichnet, dass** die Mittel zum lokalen Verabreichen eines Arzneimittels ein getrenntes Lumen in dem Katheter enthalten.

15. Medizinische Vorrichtung nach einem der vorhergehenden Ansprüche für die Verwendung bei der Behandlung eines myokardialen Infarkts oder anderer Okklusionen von Körpergefäßen.

16. Medizinische Vorrichtung nach einem der vorhergehenden Ansprüche 1 bis 14 für die Verwendung bei der Perfusion von Organen oder Geweben, die explantiert werden sollen.

## Revendications

1. Dispositif médical conçu pour l'administration locale d'un médicament comportant :
un cathéter (1) à ballonnet comportant un moyen d'administration locale d'un médicament qui est relié fonctionnellement à
au moins une pompe (4) contrôlable entraînée automatiquement,
au moins un capteur (6) de pression et
une unité (5) de commande pour commander la pompe en fonction d'un signal de pression du capteur (6) de pression,
**caractérisé en ce que**
au moins un capteur (6) de pression est placé sur la surface du ballonnet (2).

2. Dispositif médical selon la revendication 1 **caractérisé en ce que** la pompe (4) est adaptée pour pomper et/ou aspirer un fluide.

3. Dispositif médical selon la revendication 1 ou 2 **caractérisé en ce que** la pompe (4) peut être entraînée à un débit de pompage réglable et/ou une pression de pompage réglable.

4. Dispositif médical selon l'une quelconque des revendications 1 à 3 **caractérisé en ce que** la pompe est une pompe (4) de seringue.

5. Dispositif médical selon la revendication 4 **caractérisé en ce que** la pompe (4) de seringue est une pompe de seringue programmable.

6. Dispositif médical selon l'une quelconque des revendications précédentes **caractérisé en ce que** la pompe (4) entraînée automatiquement est une pompe à fluide pour pomper un médicament comportant un fluide et **en ce que** le dispositif médical comporte en outre au moins une pompe de gonflage qui est séparée et indépendante de la pompe (4) à fluide.

7. Dispositif médical selon l'une quelconque des revendications précédentes **caractérisé en ce que** le signal de pression est la pression de gonflage du ballonnet (2) et/ou la pression de perfusion d'un fluide pompé.

8. Dispositif médical selon l'une quelconque des revendications précédentes **caractérisé en ce que** le dispositif médical comporte au moins un transducteur de pression pour mesurer un paramètre physiologique et/ou un signal de pression et pour soumettre un signal correspondant à l'unité (5) de commande.

9. Dispositif médical selon la revendication 8 **caractérisé en ce que** le paramètre physiologique est choisi dans le groupe composé d'une pression sanguine systémique, une pression sanguine devant le cathéter (1) à ballonnet, une pression sanguine derrière le cathéter (1) à ballonnet, un écoulement sanguin devant le cathéter (1) à ballonnet, un écoulement sanguin derrière le cathéter (1) à ballonnet et un électrocardiogramme.

10. Dispositif médical selon l'une quelconque des revendications précédentes **caractérisé en ce que** l'unité (5) de commande commande le moment du pompage et/ou le volume de pompage.

11. Dispositif médical selon l'une quelconque des revendications précédentes **caractérisé en ce que** le moyen d'administration locale d'un médicament comporte au moins un ballonnet (2) poreux ou microporeux.

12. Dispositif médical selon l'une quelconque des revendications précédentes **caractérisé en ce que** le cathéter à ballonnet comprenant un moyen d_{'}administration locale d'un médicament comporte un revêtement sur le ballonnet (2).

13. Dispositif médical selon l'une quelconque des revendications précédentes **caractérisé en ce que** le cathéter (1) à ballonnet comporte au moins deux ballonnets.

14. Dispositif médical selon la revendication 12 **caractérisé en ce que** le moyen d'administration locale d'un médicament comporte une lumière séparée dans le cathéter.

15. Dispositif médical selon l'une quelconque des revendications précédentes destiné à être utilisé pour traiter les infarctus du myocarde ou les occlusions de vaisseaux du corps.

16. Dispositif médical selon l'une quelconque des revendications précédentes 1 à 14 destiné à être utilisé pour la perfusion d'organes ou de tissus devant être explantés.
